# NEW EUROPEAN PATENT SPECIFICATION

(11) **EP 0 246 864 B2**
(45) Date of publication and mention of the opposition decision: **23.05.2007**
(45) Mention of the grant of the patent: 13.07.1994
(21) Application number: 87304433.3
(22) Date of filing: 19.05.1987
(51) Int. Cl.: C12Q 1/68

(54) **Hybridisation probes**
Hybridisierungssonden
Sondes d'hybridation

(30) Priority: 19.05.1986 GB 8612087
(43) Date of publication of application: 25.11.1987
(73) Proprietor: BIO-RAD LABORATORIES, INC., Hercules California 94547 (US)
(72) Inventor: Carr, Francis Joseph, Little Neston South Wirral L64 0UP (GB)
(74) Representative: Helbing, Jörg

(56) References cited:
- EP-A- 0 070 685
- EP-A- 0 130 515
- EP-A- 0 185 494
- EP-A- 0 224 126
- WO-A-87/06956
- NUCLEIC ACIDS RESEARCH, vol. 14, no. 22, 1986, Oxford (GB); P.K. ROGAN, p. 9219#

## Description

The present invention relates to a method for the use of hybridisation probes, hybridisation probes therefor and kits for use in such a method. The present invention is particularly concerned with discriminating a specific base sequence from a variant base sequence and relates in particular to the advantages which may be obtained by subjecting adjacent segments of a target base sequence to hybridisation with a detectable first nucleotide probe and a second nucleotide probe.

The present invention thus relates inter alia to discriminating specific base sequences whilst ameliorating the problem of background non-specific hybridisation commonly encountered with hybridisation of short oligonucleotide probes to complex genomes.

Nucleic acid hybridisation analysis is a technique of wide applicability in the fields of biomedical research and recombinant DNA technology. Thus for example hybridisation probes are useful for detecting, monitoring, locating and isolating nucleic acids and other molecules of scientific or clinical interest Particularly useful are small oligonucleotide hybridisation probes which may for example be used to detect changes in DNA base sequences in relation to certain disease states such as phenylketonuria, alpha-antitrypsin deficiency, alpha- and beta-thalassaemia and sickle cell anaemia. These disease states are often associated with known single point.mutations in genes.

Whilst short oligonucleotide hybridisation probes are of considerable utility, they are usually associated with a high background of non specific hybridisation particularly when used for analysis of the genomes of higher organisms. This problem arises from the greater complexity of the genome. Thus for example mammals may contain of the order of a thousand-fold more DNA per cell than bacteria, and many of the nucleotide sequences may be repeated. For any given nucleotide probe therefore stable duplexes (hereinafter also referred to as hybrids) other than the desired duplex will usually be formed. This problem is particularly exacerbated by using very high oligonucleotide concentrations in order to accelerate the hybridisation process. This problem can be circumvented by using gel electrophoresis to resolve different DNA (or RNA) fragments before oligonucleotide hybridisation to ensure that a specifically hybridising fragment is resolved from non-specifically hybridising fragments. For example, Woo et al (Banbury Report. Recombinant DNA Applications to Human Disease, Cold Spring Harbor Laboratory (1983) p105-110) employ the Southern transfer technique to resolve small restriction fragments hybridising to an alpha,antitrypsin oligonucleotide probe from higher molecular weight species of DNA which also hybridise to this probe.

Oligonucleotide hybridisation analysis of complex genomes using gel electrophoresis requires the preparation of pure DNA (or RNA) molecules from the organisms, fragmentation of DNA molecules by treatment with restriction endonucleases and, in most cases, transfer of resolved DNA (or RNA) molecules from the gel to a solid support for hybridisation. The complexity of this procedure makes it unsuitable for routine diagnostic use and difficult to automate.

Simpler methods for hybridisation analysis with specifically hybridising long polynucleotide probes are exemplified by J Brandsma and G Miller, Proc. Nat. Acad. Sci.(USA) Vol 77 (1980) p6851-6855 for DNA and by T. Manser and M L Getter Proc.Natl. Acad. Sci USA Vol 81 (1984) p2470-2474 for RNA and involve directly immobilising DNA or RNA.from crude cellular lysates onto solid supports. Because of non-specific hybridisation, such direct immobilisation methods are not usually compatible with the use of oligonucleotide probes.

EP-A-0 185 494 which belongs to the state of the art in the sense of Article 54(3) EPC relates to a method of testing for the presence or absence of a target nucleotide sequence in a sample containing denatured nucleic acid. The said method specifically requires hybridization conditions in which a first probe (the diagnostic probe) only binds to the target nucleic acid if it is complementary to the diagnostic portion of the target sequence. A second probe (the contiguous probe) is complementary to a nucleotide sequence contiguous with the diagnostic portion. If the diagnostic probe is bound it will then be ligated to the contiguous probe and detected. Accordingly, if the target sequence is not identical to the diagnostic probe no binding will occur.

In addition to the above problem of background non-specific hybridisation the present invention also relates inter alia to simplifying the detection of translocations which hitherto have only been detectable by gel methods.

The present invention is thus based, at least in part, on the discovery of a method for discriminating between alternative nucleotide sequences whilst ameliorating the above-mentioned problems. The method of the present invention is thus of interest in inter alia simplifying the detection of translocations and enabling a mutation in a given base sequence to be readily detected by for example allowing meaningful analysis of directly immobilised DNA or RNA samples or alternatively DNA or RNA samples in solution. In this connection it is known that the shorter the nucleotide probe the poorer is the selectivity of hybridisation and thus correspondingly the greater is the background. Moreover the shorter the nucleotide probe the weaker is the stability of the duplex formed as measured by its melting temperature (Tm) which is the temperature of the midpoint of thermal transition. The present invention is based at least in part, on the discovery that the different hybridisation properties of a short nucleotide probe relative to a comparitively longer nucleotide probe may be exploited to advantage in discriminating between alternative nucleotide sequences, whilst ameliorating the above-mentioned problems.

The present invention is further based on the discovery of a method which substantially simplifies the hybridisation analysis thus providing a robust technique for discriminating between alternative nucleotide sequences.

Thus according to one feature of the present invention there is provided a method for discriminating between alternative nucleotide sequences, which method comprises subjecting adjacent segments of a target base sequence to hybridisation with a detectable first nucleotide probe and with a second nucleotide probe, to form a hybrid, the nucleotide sequence of the first and second probe being such that where they form a split probe hybrid with a complementary target sequence they may subsequent be linked subjecting any hybrid obtained to linkage, followed by detection of any hybrid obtained;
the DNA sequence of the detectable first nucleotide probe and of the second nucleotide probe being such that a potential mismatch in the target sequence lies between said probes;
the method being effected such that a complementary target sequence is discriminated from a target sequence with one or more non-complementary nucleotides.

Where necessary any hybrid obtained following linkage may be subjected to selective denaturing.

The presence of a linked probe may be detected by any convenient means for example selective denaturing or where one of the probes carries one member of a binding pair then by use of the other member of the binding pair. Appropriate examples of binding pairs are discussed hereinafter. It will be appreciated that one might identify either a complementary or a non-complementary target sequence by the absence of a detectable hybrid and this is included within the scope of the present invention.

It will be appreciated that hitherto discriminatory specificity has been achieved by careful manipulation of the temperature of hybridisation and washing of bound discriminatory probes. In the present invention the probes are designed to hybridise to the target sequence on either side of a potential mismatch, there being a gap, preferably a single nucleotide gap, between the probes. A gap, preferably a single nucleotide gap, is present between the two probes of the present invention which enables a method of discrimination to be effected in which incorporation of nucleotides with for example the Klenow fragment of DNA polymerase 1 or calf thymus DNA polymerase alpha to fill in the gap and allow linkage, with for example DNA ligase, will be significantly less efficient where the deoxy nucleotide triphosphate added, to for example the DNA polymerase reaction mixture is not complementary to the base residue under examination.

The present invention thus enables the hybridisation analysis to be substantial simplified and allows the production of a robust technique for discriminating between alternative nucleotide sequences. Thus for example the need to rely on the use of a hybridisation or wash within a narrow temperature range and for a specific time may be obviated thus overcoming the difficulties inherent in any technique which relies on narrow margins of error. The present invention even renders it possible in at least certain embodiments to effect the hybridisation analysis at room temperature.

The method of the present invention may for example be effected either by detection of a detectable signal, resulting from complementary hybrid formation where a complementary target sequence is present the hybrid, if necessary, being denatured prior to detection; or by failure to detect a detectable signal, resulting from selective denaturation of any hybrid formed, where a target sequence with one or more non-complementary nucleotides is present.

Thus whilst the first nucleotide probe must be detectable the second nucleotide may or may not be detectable depending on the embodiment of the method to be employed.

The term detectable is used herein to mean capable of detection. Thus the first nucleotide probe need not carry a signalling means such as a radioactive label or a non-radioactive signalling complex, although such may be present, provided that the probe may subsequently be treated to render it capable of signalling. It is thus possible to discriminate between sequences in a complex genome differing by as little as one base pair.

Where a split probe hybrid would be formed following hybridisation, with a complementary target base sequence, any hybrid obtained is subjected to a linking reaction for finking the detectable first nucleotide probe to the second nucleotide probe, any hybrid obtained being subjected to appropriate treatment whereby any hybrid present, in which linkage of the detectable first nucleotide probe to second nucleotide probe has not been effected, is denatured whereas no denaturation is effected for a perfectly complementary detectable linked oligonucletide-target sequence hybrid. Appropriate treatment may for example include selective denaturation or one of the probes may comprise one member of a binding pair. Linkage of adjacent stable hybrids of first and second nucleotide probes results in greater hybrid thermal stability for the detectable oligonucleotide hybridised to a specific target sequence as opposed to detectable oligonucleotides hybridised to non specific target sequences with no adjacent oligonucleotide probe present.

It will be appreciated that the potential variant sequence is present between the segment of the target sequence to which the detectable first nucleotide probe hybridises and the segment of the target sequence to which the second nucleotide probe hybridises.

The first or second nucleotide probe may if desired have one member of a binding pair. Generally the one member of a binding pair will be carried by, or be part of, the second nucleotide probe. The other member of the binding pair may be in solution or on a support Thus where appropriate, the second nucleotide probe linked to the detectable first nucleotide probe may be isolated on a support carrying the other member of the binding pair. Such a binding pair may for example be a protein-ligand or antigen-antibody interaction such as an avidin-biotin or dinitrophenyl-antidinitrophenyl antibody interaction.

Indeed one member of the binding pair may be a nucleotide sequence, which sequence may be a portion of the probe sequence or may be a nucleotide sequence branch on the probe. The other member of the binding pair may for example be a protein which binds to the nucleotide sequence or another nucleotide sequence to which it hybridises.

The hybridisation and/or selective denaturation is preferably effected at a temperature selected to give effective hybridisation selectivity, preferably maximum hybridisation selectivity for the specific length of the linked probe. Advantageously the hybridisation and/or selective denaturation is effected in aqueous solution at an elevated temperature suitable for selective hybridisation to a mammalian genome which for a split probe when linked is preferably above 60° C generally about 68°C. Alternatively hybridisation and/or selective denaturation of such a split probe when linked may be effected at a lower temperature in the presence of an organic solvent which is effective to destabilize the hybrid, such as a solvent containing formamide. For example where about 50% formamide is used the hybridisation and/or selective denaturation is effected at about 42°C.

In a further embodiment of the present invention there is provided a method of hybridisation probing which comprises subjecting adjacent segments of the target base sequence to hybridisation with a detectable first nucleotide probe and with a second nucleotide probe such that a split probe hybrid would be formed with a complementary target sequence, and subsequently subjecting the hybrid obtained to a linking reation whereby to link together the detectable first nucleotide probe to the second nucleotide probe.

The hybridisation or selective denaturation may be effected for example at a temperature above the melting temperature of the split or single nucleotide probe hybrid but below the melting temperature of the linked probe hybrid or in the presence of an organic solvent effective to destabilise the hybrid as discussed above. Where hybridisation is effected under selective denaturing conditions a further selective denaturing step after the hybridisation but before linking may be avoided.

The first and second nucleotide probes may be finked by any convenient means known per se such as for example by enzymatic ligation using, for example, a DNA ligase or by covalent/non-covalent linking using for example a biotin-avidin cross-link.

The detectable first nucleotide probe and/or the second nucleotide probe may initially not be capable of being linked, e.g. ligated, together until the gap(s) between them is (are) filled with DNA polymerase in the presence of the target sequence of interest.

The effect of linking the detectable first nucleotide probe and second nucleotide probe is to increase the thermal stability of the cross-linked probe hybrid obtained over the corresponding detectable probe hybrid alone. Thus for example the temperature of the linked probe hybrid may be increased to a temperature which denatures the corresponding split probe hybrids or a corresponding hybrid in which only one of the detectable first nucleotide probe and second nucleotide probes is hybridised to the target base sequence. The linked probe hybrid which includes the complementary target base sequence is thus left intact The method of the present invention thus enables one to discriminate a base sequence which is perfectly complementary to the detectable probe sequence from alternative sequences.

It will be appreciated that while the above embodiment of the invention is most useful for directing oligonucleotides to specific hybridisation sites in complex genomes in order for example, to analyse for the presence of point mutations it is also of use for analysis of other variations In complex genomes such as translocations. For analysis of translocations, It is preferred that the non-detectable second polynucleotide of a split probe will have one member of a binding pair associated therewith and will hybridise to genomic sequences adjacent to potential translocation breakpoints.

In another embodiment of the present invention, there is provided a method as hereinbefore defined wherein each of the detectable first nucleotide probe and second nucleotide probe carry a moiety such that after hybrid formation, and denaturation where appropriate, a signal is only detectable if a nucleotide sequence is obtained which carries both the moiety attached to the detectable first nucleotide probe and the moiety attached to the second nucleotide probe.

The nucleotide probes may for example be oligonucleotide probes.

The method of this embodiment might for example be effected using non-radioactive energy transfer procedures (see for example European Patent Publication No. 70685 of Standard Oil Co.) or enzyme chanelling techniques (see for example European Patent Publication No. 95087 of Syva Co.). Thus this embodiment may provide a homogenous assay for a specific target nucleotide sequence.

In another embodiment of the invention there is provided a method for discriminating between alternative nucleotide sequences in which a target sequence is subjected to hybridisation with more than two oligonucleotide probes such that where a complementary target sequence is present a split probe hybrid is obtained, the detectable first nucleotide probe being a terminal oligonucleotide probe which is detectable and the second nucleotide probe being the other terminal oligonucleotide probe which has attached thereto one member of a binding pair, individual other oligonucleotide probe(s) being hybridised separately but adjacently to a contiguous target sequence between the said terminal oligonucleotide probes, the hybrid obtained is then subjected to selective denaturation whereby to denature any oligonucleotide probe hybridised to the target sequence across a base pair mismatch, and the individual oligonucleotide probes linked to join the detectable oligonucleotide probe to the oligonucleotide probe having one member of a binding pair attached thereto to form a linked probe hybrid which is then denatured and contacted with the other member of the binding pair whereby the detectable linked probe nucleotide sequence including the said binding pair may be separated from other nucleotide sequences. Thus one terminal oligonucleotide (hybridising adjacent to a series of other oligonucleotides) for example has attached thereto a detectable signalling moiety while the other terminal oligonucleotide has attached a moeity forming part of a binding pair the moiety being effective to enable the oligonucleotide to be recovered in solution from a mixture containing other nucleotide sequences. Adjacently hybdridised oligonucleotides are linked together by appropriate treatment in order to effectively join the signalling and binding moieties via a linked probe where no oligonucleotide hybrid is denatured prior to linkage. Loss of any individual oligonucleotide hybrid results in subsequent failure to join the signalling and binding moieties. The linked probe hybrid is denatured and contacted with the other member of the binding pair which recognises one terminal oligonucleotide in the linked probe. The linked probe may thus if desired be separated from other oligonucleotides in the mixture and then analysed for the presence of the detectable moiety

Thus the association of the binding oligonucleotide with the signalling oligonucleotide is dependent on the presence, upon linking of adjacently hybridised oligonucleotides, of all adjacent oligonucleotides between the hybridised signalling oligonucleotide and the binding oligonucleotide. The binding pair effecting separation of the linked probe with an associated binding moiety may be avidin and biotin or an antigen and associated specific antibody. This embodiment provides for analysis of longer stretches of target nucleotide sequence than with just two adjacent hybridising linked or split probes as in other embodiments.

A further embodiment of the present invention comprises hybridising the detectable first nucleotide probe and second nucleotide probe to each side of a potential variant sequence in a target sequence and subsequently attempting to link the said probes, preferably by the use of DNA polymerase introducing a nucleotide(s) complementary to either the normal or the suspected variant sequence and ligation, followed by detection of any hybrid obtained. Such detection may for example be effected by subjecting any hybrid obtained to selective denaturing whereby any hybrid present, in which linkage of the detectable first nucleotide probe to the second nucleotide probe has not been effected, is denatured.

The DNA polymerase used may for example be the Klenow fragment of DNA polymerase I or calf thymus DNA polymerase alpha. Ligation is preferably effected with DNA ligase. The detectable first nucleotide probe and second nucleotide probe are preferably such that they hybridise to the target sequence whereby to leave a gap of a single nucleotide between the said probes.

Where it is desired that the nucleotide probe should carry a signal or a residue capable of producing a signal, such signals or residues may be known per se e.g. a radioactive label.

The residue which is capable of producing a non-radioisotopic signal may alternatively comprise a signalling part normally separated from the oligonucleotide by a spacer group. This spacer group may if desired be linked to the signalling part of the complex by a direct covalent link or by a protein-ligand or antigen-antibody interaction, for example, an avidin-biotin or dinitrophenyl-antidinitrophenyl antibody interaction. It will be understood that the signalling part of the residue may itself be capable of signalling or may be capable of producing a signal by interaction with an appropriate agent according to methods known per se. Thus, for example a preferred signalling part of the covalently attached residue incorporates a system for producing an enzymatically-activated production of colour change. Preferred enzyme systems involve alkaline phosphatase, acid phosphatase, beta galactosidase, luciferase, or horseradish peroxidase. Such enzyme systems are not themselves capable of signalling, but are capable of producing a signal in the presence of an appropriate substrate according to methods known per se. The signalling part of the covalently-attached residue may operate according to any conventional technique such as for example by luminescence, fluorescence or by means of colour.

In use it will generally be necessary for the nucleic acid probe to detect minute amounts of the fully complementary oligonucleotide sequence. In such circumstances it will be advantageous to incorporate within the probe a means of amplifying the signal. The amplification can be carried out by known techniques, for example using one or more of the systems described in European Patent Publications 27036 (Self), 49606 (Self), 58539 (Self) and 60123 (Self).

According to a further feature of the present invention there are provided split probe hybrids which comprise a detectable first nucleotide probe and a second nucleotide probe hybridised to adjacent segments of a target base sequence, the detectable first nucleotide probe being capable of linkage to the second nucleotide probe, characterised in that the detectable first nucleotide probe and the second nucleotide probe are hybridized to either side of a variant sequence associated with a disease state or to the corresponding normal sequence.

The non-nucleotidyl cross-link may for example be a disulphide link or a biotin-avidin link.

At least the first nucleotide probe is detectable and thus preferably carries a signal or a residue capable of producing a signal as described above.

In order to enable the method of the present invention to be effected conveniently it will generally be advantageous to incorporate the nucleotide probes in a kit and this kit is therefore regarded as a further feature of the invention.

Thus according to a further feature of the present invention there is provided a kit for discriminating between alternative nucleotide sequences which comprises a detectable first nucleotide probe and a second nucleotide probe, each probe having a nucleotide sequence, homologous to adjacent segments of a target sequence, a potential variant sequence being present between said segments; the detectable first nucleotide probe and the second nucleotide probe being such that a potential variant sequence is present between said probes, the kit additionally containing a reagent(s) for linking said probes where the probes are not so linked.

The potential variant sequence will commonly be a point mutation associated with a disease state. In such a case the kit will preferably contain a detectable first nucleotide probe and second nucleotide probe for detecting a point mutation associated with a disease state as well as a detectable first nucleotide probe and a second nucleotide probe for detecting the corresponding normal sequence in which the point mutation was absent.

It will be appreciated in this regard that certain disease states are characterised by point mutations which appear in different regions of the human DNA genome. In such a case it would be important to determine that each relevant point mutation was present or absent and thus more than one set of probes (detectable first nucleotide probe and second nucleotide probe at least) would be employed, one set of probes to detect the presence or absence of each point mutation. It would also be advantageous to include in the kit set(s) of probes for the corresponding normal or variant sequence.

The kit will also advantageously contain means for extracting DNA and/or means for immobilising DNA on a solid support.

If desired at least one of the detectable first nucleotide probe and second nucleotide probe may carry one member of a binding pair, the other member of the binding pair being present for example on a solid support provided with the kit.

The kit will also comprise appropriate buffer solutions and/or washing solutions and will contain written or printed instructions for use of the kit according to the method of the present invention.

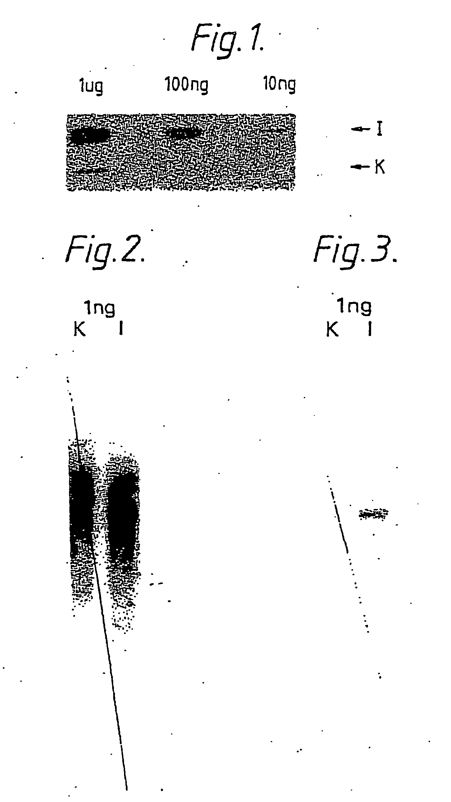

With reference to the drawings:-
Figures 1, 2 and 3 are autoradiographs in which I represents the DNA sequence of human interferon alpha₂ in the plasmid pIFS1101 as set out in Edge et al, Nucleic Acids Research Vol 11, (1983) p6419 to p6435 and K represents the DNA sequence which codes for the interferon alpha₂ analogue [Ala²⁸]IFN-alpha₂ in which the codon TCC is replaced by GCT. This analogue base sequence is herein designated as pK9.

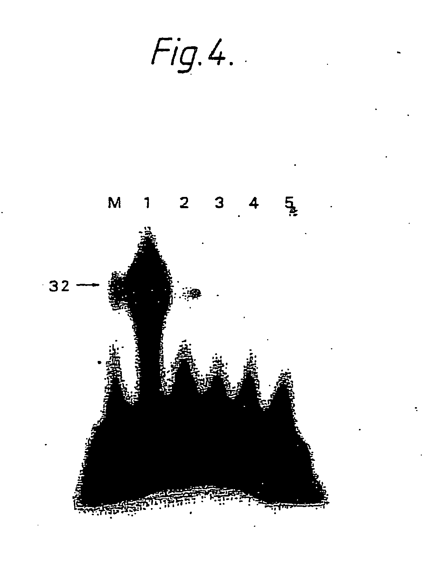

Figure 4 is an autoradiograph in which lane M shows a signal corresponding to a 32 base long ' oligonucleotide marker band, lane 1 shows a signal corresponding to a 32 base long ligation product derived from oligonucleotides 11(-1) and 13 (see hereinafter) each with a single dCTP residue incorporated at its 3' end. Lanes 2 to 5 fail to shown any signal correponding to a 32 base long oligonucleotide.

The invention is illustrated, but not limited, by the following Examples. In the Examples, unless otherwise stated, the solutions are aqueous and the % values are w/v.

The constitution of various reagents is as follows:-
SSC is 0.15M NaCl + 0.015M sodium citrate;
Kinase buffer is 0.066M Tris-HCl pH 7.6. 1mM spermidine, 0.01 M MgCl₂, 15mM dithiothreitol and 02 mg/ml bovine serum albumin (BSA);
10 x CORE BUFFER is 500 mM Tris HCl pH8, 100 mM MgCl₂. 500 mM NaCl;
10 x nick tranlation buffer is 0.5 M Tris.HCl (pH 7.2). 0.1 M MgSO₄. 1mM dithiothreitol and 500 µg/ml BSA (Pentax, fraction V):
Triton^{®}-X-100 is a polyoxyethylene ether surface active compound;
Ficoll is a non-ionic synthetic polymer of sucrose having a molecular weight of approximately 400,000 in dialysed and lyophilised form;
Nonidet P-40 is an octyl phenol-ethylene oxide condensate containing an average of 9 moles ethylene oxide per molecule;
Denhardt's reagent is 0.2 g/l Ficol 400,000, 0.2 g/l polyvinyl pyrrolidone (PVP) and 02 g/l bovine albumin (BA);
PBS- (phosphate buffered saline) is 0.01 M sodium phosphate pH 7.4 and 0.13M NaCl:
SSPE is 10 mM sodium phosphate pH7, 0.18M NaCl and 1 mM EDTA;

The following contractions are used:-

| | |
|---|---|
| DNA | deoxyribonucleic acid |
| tRNA | transfer ribonucleic acid |
| EDTA | ethylenediaminetetraacetic acid |
| PBS | phosphate buffered saline |
| SDS | sodium dodecyl sulphate |
| 2 x SSC | double concentration SSC |
| 6 x SSC | six times concentration of SSC |
| 20 x SSC | twenty times concentration of SSC |
| BSA | bovine serum albumin |
| BA | bovine albumin |
| PVP | polyvinyl pyrrolidone |
| Tris | tris(hydroxymethyl)aminomethane |
| 10 x Core | buffer ten times the concentration of CORE BUFFER |
| ATP ' | adenosine triphosphate |
| NP40 | Nonidet P.40 |
| 10 x nick translation buffer | ten times the concentration of nick translation buffer |
| | The following are trade marks |
| | Minifold |
| | Triton-X-100 |
| | CORE BUFFER |
| | Ficoll |

### EXAMPLE 1 (Reference Example)

Plasmids used for oligonucleotide hybridisation analysis were plFS1101 (Edge et al., Nucleic Acids Research. Vol 11 (1983) p6419 to 6435) and a derivative thereof designated herein as pK9 in which the codon GCT coding for alanine is present at amino acid position 28 in place of the codon TCC which codes for serine in HulFN-alpha₂. Aliquots of 1 µg, 100 ng and 10 ng were diluted to 153 ul in water. To these were added 30 µl 2MNaOH, 17 ul 1M Tris.HCl pH7.4 and 100 µl 20 x SSC (SSC is 0.15M NaCl, 0.015M sodium citrate). Samples were heated to 100°C for 10 minutes, then placed on ice and 70 µl 1M Tris.HCl pH7 was added. Dilutions of plasmid DNA were filtered onto BA 85 nitrocellulose filters (Schleicher and Schuell Keene, N.H., USA) using a Schleicher and Schuell Minifold II apparatus according to the manufacturer's instructions. The nitrocellulose paper was cut to fit the Minifold apparatus and wetted by floating on 2 x SSC before mounting in the apparatus. DNA samples were then applied to the wells and filtered at a rate of approximately 0.5 ml per minute. The nitrocellulose was then air dried for 10 minutes and baked at 80°C for 2 hours in vacuo. The oligonucleotides 11 and 13 (Edge et al., see above) corresponding to the sequence of the synthetic interferon alpha₂ gene In pIFS1101 were used for hybridisation. Oligonucleotides 11 and 13 have the following DNA sequences:
Oligonucleotide 11 5' CGTATCTCCCTGTTC 3'
Oligonucleotide 13 5' TCCTGTCTGAAAGAC 3'

Oligonucleotide 13 was labelled with ³²P as follows: to 400 ng of oligonucleotide in 18.5 µl H₂O was added 20 µl ³²P gamma ATP (Adenosine 5'-[³²P]triphosphate, triethylammonium salt in aqueous solution, approx. 3000 Ci/nmol, 10 µCi/µl. Amersham International), 4.5 µl 10 x kinase buffer (10 x buffer = 0.66M Tris. HCl pH7.6, 10mM spermidine, 0.1M MgCl₂, 150nM dithiothreitol and 2 mg/ml bovine serum albumin (BSA, Nucleic Acid Enzyme Grade, Bethesda Research Laboratories)) and 2 µl T4 polynucleotide kinase (5 units/µl, Boehringer Mannheim). After 1 hour at 37°C, half of the sample was applied to a 4 ml column of Biogel P2 (Biorad; pre-equilibrated with 10mM Tris.HCl pH8, 1mMEDTA). The ³²P labelled nucleotide was eluted in the void volume of the elution buffer comprising 10mM Tris.HCl pH8, 1mM EDTA. 100 ng of ³²P labelled oligonucleotide probe was used for hybridisation together with 100 ng unlabelled oligonucleotide 11. Nitrocellulose filters onto which piFS1101 or pK9 DNA had been immobilised were prehybridised for 2 hours at 68°C in 5 x Denhardt's reagent (1 g/l Ficoll, 1 g/l polyvinylpyrrolidone, 1 g/l BA (bovine albumin, Fraction V, Miles laboratories), 5 x SSC, 50mM sodium phosphate pH7, 1% glycine, 0.1% sodium dodecyl sulphate (SDS) and 100 µg sonicated denatured herring sperm DNA (Sigma). Hybridisations were performed in 2 ml of 5 x SSC, 0.5% NP40 (BDH) and 250 µg/ml tRNA (Sigma, type X-S) with 100 ng of either oligonucleotides 11 and 13 added together or, as a control, 100 ng of oligonucleotide 13 added alone. Hybridisations were performed overnight at room temperature. Filters were then washed in 6 x SSC, 0.06% sodium pyrophosphate and 20mM sodium phosphate buffer, pH7 for 5 minutes at room temperature and, after changing the wash buffer, for a further 3 minutes at room temperature. Filters were then treated with phosphate buffered saline (PBS: 0.01 M sodium phosphate pH7.4, 0.13M NaCl) containing 2% BA and 0.1% Triton-X-100(BDH) for 30 minutes at 16° C. Filters were then rinsed 5 times with PBS and 2% BA to remove the Triton-X-100. For cross-linking of hybridised probes, filters were treated with 66mM Tris.HCl pH7.6. 5mMMgCl₂, 5mM dithiothreitol, 1 mM ATP, 500 µg/ml BSA and 0.3 U/µl T4 DNA ligase (Boehringer, 5U/ul). Incubation was for 80 minutes at 16° C. Filters were then washed at 60° C for 20 minutes in 6 x SSC, 0.06% sodium pyrophosphate and 20mM sodium phosphate pH7. The filters were then wrapped in Saran wrap and exposed to autoradiographic film for 2 days. Figure 1 shows that after the final wash at 60° C, an appreciable autoradiographic signal was observed only for the plasmid pIF51101 (I) where hybridised oligonucleotides 11 and 13 had been cross linked by ligation. A background signal corresponding to immobilised pK9 (K) was observed, after hybridisation and cross linking of oligonucleotides 11 and 13. This background signal represented about 1% of the corresponding signal obtained for p1FS1101 and resulted from the minimal hybridisation of oligonucleotide 13 because of hybrid mismatches with the corresponding region of the plasmid pK9. Thus adjacent hybridised oligonucleotide probes were shown by this example to cross link efficiently by ligation on nitrocellulose filters. Moreover, with the experimental conditions described the ligation time could be reduced to 5 minutes without any loss of autoradiographic signal intensity.

### EXAMPLE 2 (Reference Example)

High molecular weight DNA was isolated from the lymphoblastoid line Daudi using the method of Blin and Stafford (Nucleic Acids Research, Vol.3 (1976) p2303). 200 µg of Daudi DNA was diluted into 320 µl H₂O and to this was added 40 µl of 10 x CORE BUFFER (Bethesda Research Laboratories) and 40 µl EcoRI (50 units/µl Northumbria Biologicals, Cramlington, Northumberland, UK). Following an incubation at 37°C for 3 hours, the solution was extracted with an equal volume of phenol/chloroform (1:1), then with 1 volume of chloroform and finally three times with 1 volume of H₂O saturated ether. The DNA was adjusted to 0.3M sodium acetate and precipitated with 2 volumes of ice cold ethanol. The DNA pellet was washed in cold 70% ethanol and the pellet, dried and redissolved in 100 µl H₂O.

3 ug of the plasmids pIFS1101 and pK9 (see Example 1) were dissolved in 17 µl H₂O and to this was added 2 µl 10 x CORE BUFFER and 1 µl EcoR1 (see above). The mixture was incubated for 3 hours at 37°C and was followed by solvent extractions and EtOH precipitations as for the Daudi DNA above. The dried pellets were redissolved in 10 µl H₂O.

10 µg of EcoR1 digested Daudi DNA was mixed with 1 ng aliquots of either pIFS1101 or pK9 and subjected to gel electrophoresis on a 0.5% horizontal agarose gel as described in Maniatias et al (Molecular Cloning, A Laboratory Manual: Cold Spring Harbor Laboratory. 1982). Following electrophoresis, DNA was either transferred onto nitrocellulose filters by the Southern transfer procedure (Maniatis et al) for subsequent hybridisation or alternatively. DNA was probed directly within dried agarose gels prepared as described by Studencki and Wallace (DNA, volume 3, p7 to 15 (1984)). For DNA transferred onto nitrocellulose filters hybridisation was as described in Example 1 except that the hybridisation temperature was 32°C using 100 ng oligonucleotide 11 and 100 ng ³²P labelled oligonucleotide 13 as probes. The filters were washed for 30 minutes at room temperature in 5 x SSC, 0.06% sodium pyrophosphate and 20mM sodium phosphate pH7 and then at 40°C, for 45 minutes to remove most of the ³²P labelled oligonucleotide associated with the pK9 plasmid DNA. The filters were then exposed at -70°C to Kodak^{®} X-Omat AR Film using intensifying screens. After this 40°C wash, a background signal derived from hybridisation to Daudi cell DNA was still evident as shown in Figure 2.

Dried agarose gels were hybridised directly with 100 ng of both oligonucleotides 11 and ³²P labelled 13 in 50 mM sodium phosphate pH7, 0.9 M NaCl, 5 mM EDTA, 0.3% SDS and 10 µg/ml E.coli DNA at 32°C overnight. The dried gels were subsequently washed in 2 x SSPE (SSPE is 10mM sodium phosphate pH7, 0.18M NaCl, 1mM EDTA) and 0.1% SDS at room temperature for 30 minutes and then at 40°C for 15 minutes in the same washing buffer. The result was similar to that observed on nitrocellulose filters (Figure 2). In order to remove background due to hybridisation to Daudi cell DNA, oligonucleotides 11 and 13 hybridise to DNA immobilised either In nitrocellulose or dried gels were cross-linked by ligation, as described in Example 1, and finally washed at 60° C for 15 minutes as above. This resulted in a single band corresponding to 1 ng of plasmid pIFS1101 with little observable background and with no signal evident corresponding to pK9 DNA as shown in Figure 3 for DNA immobilised on nitrocellulose.

### EXAMPLE 3

The plasmid DNA used for analysis was plFS1101 (Edge et al., Nucleic Acids Research, vol. 11 (1983) p6419 to 6435). 2 µg of pIFS1101 plasmid DNA was digested with EcoRI (20 units, Bethesda Research Laboratories) in a reaction volume of 20 µl for 2 hours using conditions recommended by the enzyme supplier. The reaction mixture was extracted once with phenol: chloroform (1:1) and once with chloroform before adding 2 µl 3M sodium acetate pH 5.2 and 45 µl ethanol. The DNA was precipitated at -20°C overnight, washed in 70% EtOH and dried prior to resuspension in 30 µl H₂O.

The oligonucleotides used for analysis were designated 11 (-1) and 13 where oligonucleotide 13 was used to construct at the synthetic interferon alpha₂ gene in plFS1101 and oligonucleotide 11(-1) is a derivative of oligonucleotide 11 used for gene construction with a sequence shifted 5' by 1 base. Thus the sequences of the oligonucleotides were as follows:-
Oligonucleotide 11(-1) 5'CCGTATCTCCCTGTT3'
Oligonucleotide 13 5'TCCTGTCTGAAAGAC3'

Oligonucleotide 13 was 5' end labelled with ³²P using polynucleotide kinase as described for synthetic linkers (forward reaction) in Molecular Cloning, a Laboratory Manual (Editors: Maniatis, Fritsch and Janbrook, Cold Spring Harbor). The kinase reaction was stopped by heating to 70°C for 10 minutes followed by 2 successive phenol extractions. The solution was then extracted with ether and excess ether was blown off.

4 µl aliquots of EcoRl digested pIFS1101 DNA were added to 50 µl aliquots of a solution of 10 x SSC, 1% NP40 and 0.5 µg/ml tRNA. 1 pmole aliquots of ³²P labelled oligonucleotide 13 either with or without 1 pmole of oligonucleotide 11(-1) was added and the solutions diluted to 100 µl with water. The solutions were heated to 100°C for 5 minutes and then placed in a water bath at 39°C for 2 hours in order for the oligonucleotides to hybridise to the target pIFS1101 DNA.

DNA samples were dissolved in 10 µl H₂O to which was added 1 µl of either 2nM dCTP or 2nM dGTP (PL Biochemicals). Then 2.5 µl of 10 x nick translation buffer (see Molecular Cloning, a Laboratory Manual referred to above) 10 x buffer is 0.5 M Tris.HCl (pH 7.2), 0.1 M MgSO₄ 1mM dithiothreitol and 500 µg/ml BSA (Pentax. fraction V) was added and the solutions were diluted with water to 25µl. 2 Units of the Klenow fragment of DNA polymerase I (Boehringer) was added and the solutions incubated at room temperature for 30 minutes. 1.5 µl 10 x nick translation buffer was added together with 4 µl 10 mM ATP and 7.5 µl H₂O. 2 units of T4 DNA ligase (Bethesda Research Laboratories) were added and the solutions were incubated at 14°C for 4 hours.

2 µl of reaction mixtures were analysed on an 8% native polyacrylamide gel. Prior to loading, samples were heated to 100°C for 5 minutes and rapidly cooled on ice before adding 1 µl of gel loading buffer (0.25% bromophenol blue, 0.25% xylene cyanol and 15% Ficoll type 400). Figure 4 shows the results of this gel analysis. An autoradiographic signal corresponding to a 32 base long oligonucleotide marker band (lane M) was observed only in lane 1 where the polymerase reaction had included dCTP prior to ligase treatment. Other samples included' polymerase treatment with dCTP present but without ligase treatment (lane 2) or without oligonucleotide 11 (-1) (lane 5). Inclusion of dGTP in place of dCTP in the polymerase step either with ligase treatment (lane 3) or without ligase treatment (lane 4) did not result in an autoradiographic signal in the 32 base position.

These results indicate that production of the 32 base long ligation product derived from oligonucleotide 11(-1) and 13 requires inclusion of the correct deoxynucleotide triphosphate (dCTP) for filling in the single base pair gap opposite a G residue between the individual hybridised oligonucleotides. Inclusion of the incorrect nucleotide (e.g. dGTP) or, by inference, susbtitution of the G residue in the gap by an alternative residue, precludes the formation of the 32 base long ligation product Thus this constitutes a diagnostic test for the presence or absence of a specific nucleotide in a target DNA sequence.

### Materials and Methods

Oligonucleotides were synthesised by the improved solid phase phosphotriester method (Gait, M J et al (1980) Nucleic Acids Research 8, 1081-1096 and Markham, A F et al (1980) Nucleic Acid Research 8, 5193-5205) and purified by high performance liquid chromatography initially on Parisil 10-SAX anion-exchange columns (Jones Chromatography, Glamorgan or Whatman Ltd) then on C₁₈ µBondapak reverse phase columns (Waters Associates Inc.) as described (Newton, C R et al (1983) Anal. Biochem. 129, 22-30).

Sequencing of 5' ³²P radiolabelled oligonucleotides were as described by Maxam and Gilbert (Maxam, A M et al(1977) Proceeding of the National Academy of Sciences, USA 74, 560-564) for the cytosine plus thymine, adenine, and guanosine greater than adenine cleavages and as described by Rubin and Schmid (Rubin, C M et al (1980) Nucleic Acids Research 8, 4613-4619) for the partial cytosine. specific cleavage with hydroxylamine hydrochloride.

T4 DNA ligase was from Boehringer Mannheim GmbH and the unit definition used throughout is that according to Weiss et al (Weiss, B et al (1968) Journal of Biological Chemistry 243, 4543-4555). One unit being the enzyme activity which exchanges at 37°C In mole ³²PPI into Norit absorbable material within 20 minutes. Ligase reactions were canid out in 66 mM Tris-HCl (pH7.6), 6.6 mM MgCl₂, 10 mM dithiothreitol and 0.4 mM-ATP.

T4 polynucleotide kinase was from New England Biolabs, the unit definition being the enzyme activity producing 1 nmole of acid insoluble ³²P in 30 minutes at 37°C. Kinase reactions were carried out in 50 mM Tris-HCl (pH 9.0), 10 mM MgCl₂, 20 mM dithiothreitol, 0.1 mM spermidine, 0.1 mM EDTA. The ATP concentration was variable depending on the subsequent use of phosphorylated oligonucleotide.

Adenosine 5' -[γ⁻³²P] triphosphate, triethylammonium salt (³²P ATP) was from Amersham International. 1mCi per ml in aqueous ethanol with specific activity>5000 Ci/mmol.

Autoradiography was at room temperature or at -70°C using Kodak XR-5 film with Ilford fast tungstate intensifying screens [Laskey. R A et al (1977) Febs Letters 82, 314-341].

Electrophoresis was through 20% acrylamide (19.33% acrylamide, 0.67% bisacrylamide) gels containing 7M Urea, 50 mM Tris-borate (pH B.3) 1 mM EDTA unless otherwise stated.

Electrophoresis samples were either resuspended or diluted with 80% formamide. 10 mM NaOH. 1 mM EDTA, 0.1 % bromophenol blue. Samples were heat denatured at 100°C for 2 minutes then rapidly cooled on ice prior to electrophoresis. Electrophoresis was across a potential gradient of 40V/cm till the bromophenol blue had migrated 15 cm from the origin unless stated otherwise.

³²P labelled oligonucleotides were purified by polyacylamide gel electrophoresis and DNA was recovered from gels by electroelution into dialysis bags containing 10 mM Tris-borate (pH 8.3), 0.2 mM EDTA (1 ml) across a potential gradient of 16V/cm for 45 minutes after which the polarity was reversed for 20 seconds. Electroeluates were concentrated by successive butanol extractions to a voldume of approximately 500 µl, extracted with phenol-chloroform [buffered with 0.5 M Tris-HCI (pH 7.5) then extracted with water] (500 µl). Aqueous phases were extracted with ether (3 x 1 ml), further concentrated by butanol extraction to approximately 20 µl, dried in vacuo and ethanol precipitated. Ethanol precipitation was accomplished by resuspending the DNA in 0.3 M sodium acetate (pH 5.6. 250µl then addition and mixing of ethanol (800 µl), chilling at -70°C for 90 minutes or longer followed by centrifuging for 15 minutes in an Eppendorf microcentrifuge. The pellets were dried in vacuo, resespended in water (500 µl).

Cerenkov radiation was determined using an Intertechnique SL4000 Liquid Scintillation Counter with on fine M1750 silent 700 printer. Electroeluates were transferred to siliconised 750 ml microcentrifuge tubes placed in 20 µl standard plastic counting vials (Packard). Samples were allowed to dark adapt for 10 minutes then Cerenkov radiation determined by setting the Liquid Scintillation spectrometer to count for tritium. Bands from gels wee excised, placed directly into plastic counting vials and Cerenkov radiation was determined as described for electroeluates. Quenching of Cerenkov radiation in gel bends relative to the same amount of ³²P-labelled oligonucleotide in solution could thus be allowed for.

## Claims

1. A method for discriminating between alternative nucleotide sequences, which method comprises subjecting adjacent segments of a target base sequence to hybridisation with a detectable first nucleotide probe and with a second nucleotide probe, to form a hybrid, the nucleotide sequence of the first and second probe being such that where they form a split probe hybrid with a complementary target sequence they may subsequently be linked, subjecting any hybrid obtained to linkage, and detection of any hybrid obtained;
the DNA sequence of the detectable first nucleotide probe and of the second nucleotide probe being such that a potential mismatch in the target sequence lies between said probes;
the method being effected such that a complementary target sequence is discriminated from a target sequence with one or more non-complementary nucleotides.

2. A method as claimed in claim 1, wherein the detectable first nucleotide probe and second nucleotide probe are hybridised to each side of a potential variant sequence in a target sequence the said probes being subjected to a linkage-reaction which comprises introducing a nucleotide(s) complementary to either the normal or the suspected variant sequence and subjecting the nucleotide(s) to ligation; followed by detection of any hybrid obtained.

3. A method as claimed in claim 2, wherein detection is effected by subjecting any hybrid obtained to selective denaturing whereby any hybrid present, in which linkage of the detectable first nucleotide probe to the second nucleotide probe has not been effected, is denaturated.

4. A method as claimed in claim 2 or claim 3, wherein the introduction of the said nucleotide(s) is effected by the use of DNA polymerase.

5. A method as claimed in any one of claims 2 to 4, wherein the detectable first nucleotide probe and second nucleotide probe are such that they hybridise to the target sequence whereby to leave a gap of a single nucleotide between the said probes.

6. A method as claimed in claim 1, in which a split probe would be formed following hybridisation, with a complementary target base sequence, any hybrid obtained being subjected to a linking reaction for linking the detectable first nucleotide probe to the second nucleotide probe, and the hybrid thus obtained being subjected to appropriate treatment whereby any hybrid present, in which linkage of the detectable first nucleotide probe to second nucleotide probe has not been effected, is denaturated whereas no denaturation is effected for a perfectly complementary detectable linked oligonucleotide-target sequence hybrid.

7. A method as claimed in claim 1, wherein each of the detectable first nucleotide probe and second nucleotide probe carry a moiety such that after hybrid formation, and denaturation where appropriate, a signal is only detectable if a nucleotide sequence is obtained which carries both the moiety attached to the detectable first nucleotide probe and the moiety attached to the second nucleotide probe.

8. A split probe hybrid comprising a detectable first nucleotide probe and a second nucleotide probe hybridised to adjacent segments of a target base sequence, the detectable first nucleotide probe being capable of linkage to the second nucleotide probe, **characterised in that** the detectable first nucleotide probe and the second nucleotide probe are hybridised to either side of a variant sequence associated with a disease state or to the corresponding normal sequence.

9. A kit for discriminating between alternative nucleotide sequences according to the method of claim 1, which comprises a detectable first nucleotide probe and a second nucleotide probe, each probe having a nucleotide sequence homologous to adjacent segments of a target sequence, a potential variant sequence being present between said segments; the detectable first nucleotide probe and the second nucleotide probe being such that a potential variant sequence is present between said probes, the kit additionally containing a reagent(s) for linking said probes.

10. A kit as claimed in claim 9, which contains a detectable first nucleotide probe and second nucleotide probe for detecting a point mutation associated with a disease state as well as a detectable first nucleotide probe and a second nucleotide probe for detecting the corresponding normal sequence in which the point mutation was absent, wherein at least one of said first and second nucleotide probe carries one member of a binding pair.

## Patentansprüche

1. Verfahren zur Diskriminierung zwischen alternativen Nucleotidsequenzen, wobei das Verfahren umfasst: das Hybridisieren benachbarter Segmente einer Targetbasensequenz mit einer nachweisbaren ersten Nucleotidsonde und mit einer zweiten Nucleotidsonde zur Bildung eines Hybrids, wobei die Nucleotidsequenz der ersten und der zweiten Sonde derart sind, dass, falls sie ein Splitsondenhybrid mit einer komplementären Targetsequenz bilden, sie anschließend miteinander verknüpft werden können, das Verknüpfen eines gegebenenfalls erhaltenen Hybrids und den Nachweis eines gegebenenfalls erhaltenen Hybrids;
wobei die DNA-Sequenz der nachweisbaren ersten Nucleotidsonde und der zweiten Nucleotidsonde derart sind, dass eine potentielle Fehlpaarung in der Targetsequenz zwischen diesen Sonden liegt;
wobei das Verfahren so durchgeführt wird, dass eine komplementäre Targetsequenz von einer Targetsequenz mit einem oder mehreren nichtkomplementären Nucleotiden diskriminiert wird.

2. Verfahren nach Anspruch 1, wobei die nachweisbare erste Nucleotidsonde und die zweite Nucleotidsonde an jeder Seite einer potentiellen Varianzsequenz in einer Targetsequenz hybridisiert werden, wobei diese Sonden einer Verknüpfungsreaktion unterzogen werden, welche die Einführung eines oder mehrerer Nucleotide, die entweder zu der normalen oder der vermuteten Varianzsequenz komplementär sind, und die Ligierung des bzw. der Nucleotide umfasst; gefolgt vom Nachweis eines gegebenenfalls erhaltenen Hybrids.

3. Verfahren nach Anspruch 2, wobei der Nachweis durch selektive Denaturierung eines gegebenenfalls erhaltenen Hybrids durchgeführt wird, wodurch jedes vorhandene Hybrid, in welchem die Verknüpfung der nachweisbaren ersten Nucleotidsonde mit der zweiten Nucleotidsonde nicht durchgeführt wurde, denaturiert wird.

4. Verfahren nach Anspruch 2 oder Anspruch 3, wobei die Einführung des bzw. der Nucleotide durch Verwendung von DNA-Polymerase durchgeführt wird.

5. Verfahren nach einem der Ansprüche 2 bis 4, wobei die nachweisbare erste Nucleotidsonde und die zweite Nucleotidsonde derart sind, dass sie an die Targetsequenz hybridisieren, wodurch ein Lücke von einem einzelnen Nucleotid zwischen diesen Sonden zurückbleibt.

6. Verfahren nach Anspruch 1, wobei eine Splitsonde im Anschluss an die Hybridisierung mit einer komplementären Targetbasensequenz gebildet würde, wobei ein gegebenenfalls erhaltenes Hybrid einer Verknüpfungsreaktion zum Verknüpfen der nachweisbaren ersten Nucleotidsonde mit der zweiten Nucleotidsonde unterzogen wird und das so erhaltene Hybrid einer geeigneten Behandlung unterzogen wird, wodurch gegebenenfalls vorhandenes Hybrid, in dem die Verknüpfung der nachweisbaren ersten Nucleotidsonde an die zweite Nucleotidsonde nicht erfolgte, denaturiert wird, während keine Denaturierung für ein perfekt komplementäres, nachweisbares, verknüpftes Oligonucleotid-Targetsequenzhybrid erfolgt.

7. Verfahren nach Anspruch 1, wobei die nachweisbare erste Nucleotidsonde und die zweite Nucleotidsonde jeweils eine solche Einheit tragen, dass nach der Hybridbildung und gegebenenfalls Denaturierung ein Signal nur nachweisbar ist, wenn eine Nucleotidsequenz erhalten wird, die sowohl die an die nachweisbare erste Nucleotidsonde gebundene Einheit als auch die an die zweite Nucleotidsonde gebundene Einheit trägt.

8. Splitsondenhybrid, umfassend eine nachweisbare erste Nucleotidsonde und eine zweite Nucleotidsonde, hybridisiert an angrenzende Segmente einer Targetbasensequenz, wobei die nachweisbare erste Nucleotidsonde zur Verknüpfung mit der zweiten Nucleotidsonde fähig ist, **dadurch gekennzeichnet, dass** die nachweisbare erste Nucleotidsonde und die zweite Nucleotidsonde an beide Seiten einer mit einem Krankheitszustand assoziierten Varianzsequenz oder an die entsprechende Normalsequenz hybridisiert sind.

9. Kit zur Diskriminierung zwischen alternativen Nucleotidsequenzen nach dem Verfahren nach Anspruch 1, welcher eine nachweisbare erste Nucleotidsonde und eine zweite Nucleotidsonde umfasst, wobei jede Sonde eine zu angrenzenden Segmenten einer Targetsequenz homologe Nucleotidsequenz hat, wobei eine potentielle Varianzsequenz zwischen diesen Segmenten vorhanden ist; die nachweisbare erste Nucleotidsonde und die zweite Nucleotidsonde derart sind, dass eine potentielle Varianzsequenz zwischen diesen Sonden vorhanden ist, wobei der Kit zusätzlich ein oder mehrere Reagentien zum Verknüpfen dieser Sonden enthält.

10. Kit nach Anspruch 9, der eine nachweisbare erste Nucleotidsonde und eine zweite Nucleotidsonde zum Nachweisen einer Punktmutation, die mit einem Krankheitszustand assoziiert ist, sowie eine nachweisbare erste Nucleotidsonde und eine zweite Nucleotidsonde zum Nachweisen der entsprechenden Normalsequenz, in der die Punktmutation fehlt, enthält, wobei wenigstens entweder die erste oder die zweite Nucleotidsonde einen Partner eines Bindungspaars trägt.

## Revendications

1. Procédé pour distinguer entre deux séquences de nucléotides, consistant à soumettre des segments adjacents d'une séquence de base cible à une hybridation avec une première sonde de nucléotides détectable et une seconde sonde de nucléotides, pour former un hybride, la séquence de nucléotides de la première et de la seconde sondes étant telle qu'à l'endroit où elles forment un hybride de sondes scindé avec une séquence cible complémentaire, elles peuvent ensuite être liées, à soumettre tout hybride obtenu à une liaison et à détecter tout hybride éventuellement obtenu :
la séquence d'ADN de la première sonde de nucléotides détectable et de la seconde sonde de nucléotides étant telle qu'une erreur d'appariement potentielle dans la séquence cible réside entre ces sondes ;
le procédé étant effectué en distinguant une séquence cible complémentaire d'une séquence cible avec un ou plusieurs nucléotides non complémentaires.

2. Procédé selon la revendication 1, dans lequel la première sonde de nucléotides détectable et la seconde sonde de nucléotides sont hybridées à chaque côté d'une séquence variante potentielle d'une séquence cible, ces sondes étant soumises à une réaction de liaison qui consiste à introduire un ou plusieurs nucléotides complémentaires soit à la séquence normale, soit à la séquence variante soupçonnée et à soumettre le ou les nucléotides à une ligature ; puis à détecter tout hybride obtenu.

3. Procédé selon la revendication 2, dans lequel la détection est effectuée en soumettant tout hybride obtenu à une dénaturation sélective de sorte que tout hybride présent, dans lequel la liaison de la première sonde de nucléotides détectable à la seconde sonde de nucléotides n'a pas été effectuée, est dénaturé.

4. Procédé selon la revendication 2 ou 3, dans lequel l'introduction de ce ou ces nucléotides est effectuée en utilisant de l'ADN polymérase.

5. Procédé selon l'une quelconque des revendications 2 à 4, dans lequel la première sonde de nucléotides détectable et la seconde sonde de nucléotides sont telles qu'elles s'hybrident à la séquence cible de manière à laisser une brèche d'un seul nucléotide entre ces sondes.

6. Procédé selon la revendication 1, dans lequel une sonde scindée serait formée après l'hybridation avec une séquence de bases cible complémentaire, tout hybride obtenu étant soumis à une réaction de liaison pour lier la première sonde de nucléotides détectable à la seconde sonde de nucléotides, l'hybride ainsi obtenu étant soumis à un traitement approprié dans lequel tout hybride présent, dans lequel la liaison de la première sonde de nucléotides détectable à la seconde sonde de nucléotides n'a pas été effectuée, est dénaturé, tandis qu'aucune dénaturation n'est effectuée pour un hybride oligonucléotide lié détectable - séquence cible parfaitement complémentaire.

7. Procédé selon la revendication 1, dans lequel la première sonde de nucléotides détectable et la seconde sonde de nucléotides portent chacune une partie telle qu'après formation de l'hybride et dénaturation si celle-ci est appropriée, un signal n'est détectable que si l'on obtient une séquence de nucléotides qui porte à la fois la partie fixée à la première sonde de nucléotides détectable et la partie fixée à la seconde sonde de nucléotides.

8. Hybride de sonde scindé comprenant une première sonde de nucléotides détectable et une seconde sonde de nucléotides hybridée à des segments adjacents d'une séquence de base cible, la première sonde de nucléotides détectable étant capable de se lier à la seconde sonde de nucléotides, **caractérisé en ce que** la première sonde de nucléotides détectable et la seconde sonde de nucléotides sont hybridées à l'un et l'autre côté d'une séquence variante associée à un état pathologique ou à la séquence normale correspondante.

9. Nécessaire pour distinguer entre deux séquences de nucléotides selon le procédé de la revendication 1, qui comprend une première sonde de nucléotides détectable et une seconde sonde de nucléotides, chacune des sondes ayant une séquence de nucléotides homologue aux segments adjacents d'une séquence cible, une séquence variante potentielle étant présente entre lesdits segments, la première sonde de nucléotides détectable et la seconde sonde de nucléotides étant telles qu'une séquence variante potentielle est présente entre ces sondes, le nécessaire contenant en outre un ou plusieurs réactifs pour lier ces sondes.

10. Nécessaire selon la revendication 9, qui contient une première sonde de nucléotides détectable et une seconde sonde de nucléotides pour détecter une mutation ponctuelle associée à un état pathologique ainsi qu'une première sonde de nucléotides et une seconde sonde de nucléotides pour détecter la séquence normale correspondante dans laquelle la mutation ponctuelle était absente, dans lequel au moins une desdites première et seconde sondes de nucléotides porte un élément d'une paire de liaison.
